# EUROPEAN PATENT SPECIFICATION

(11) **EP 2 515 866 B1**
(45) Date of publication and mention of the grant of the patent: **19.02.2014**
(21) Application number: 10803219.4
(22) Date of filing: 22.12.2010
(51) Int. Cl.: A61K 9/00, A61K 31/593, A61K 47/10, A61K 47/14, A61K 47/22, A61K 9/06

(54) **PHARMACEUTICAL COMPOSITION COMPRISING SOLVENT MIXTURE AND A VITAMIN D DERIVATIVE OR ANALOGUE**
PHARMAZEUTISCHE ZUSAMMENSETZUNG MIT EINER LÖSUNGSMITTELMISCHUNG UND EINEM VITAMIN-D-DERIVAT ODER -ANALOGON
COMPOSITION PHARMACEUTIQUE COMPRENANT UN MÉLANGE DE SOLVANTS ET UN DÉRIVÉ DE LA VITAMINE D OU ANALOGUE

(30) Priority: 22.12.2009 WO PCT/DK2009/000264; 07.01.2010 US 293108 P
(43) Date of publication of application: 31.10.2012
(73) Proprietor: Leo Pharma A/S, 2750 Ballerup (DK)
(72) Inventor: PETERSSON, Karsten, DK-2750 Ballerup (DK)
(74) Representative: Thalsoe-Madsen, Kine Birgit
(86) International application number: PCT/DK2010/000184
(87) International publication number: WO 2011/076209

(56) References cited:
- WO-A1-00/64450
- WO-A1-2006/134272
- WO-A2-2008/065316

## Description

### FIELD OF INVENTION

The present invention relates to a topical pharmaceutical composition comprising a biologically active vitamin D derivative or analogue, namely calcipotriol, dissolved in a triple solvent mixture, and its use in the treatment of dermal diseases and conditions.

### BACKGROUND OF THE INVENTION

Psoriasis is a chronic inflammatory skin disease that manifests as erythematous, dry, scaling plaques resulting from hyperkeratosis. The plaques are most often found on the elbows, knees and scalp, though more extensive lesions may appear on other parts of the body, notably the lumbosacral region. The most common treatment of mild to moderate psoriasis involves topical application of a composition containing a corticosteroid as the active ingredient. While efficacious, corticosteroids have the disadvantage of a number of adverse effects such as skin atrophy, striae, acneiform eruptions, perioral dermatitis, overgrowth of skin fungus and bacteria, hypopigmentation of pigmented skin and rosacea.

For many years, however, an advantageous non-steroidal treatment of psoriasis has consisted in topical treatment with the vitamin D analogue compound, calcipotriol, formulated in an ointment composition (marketed as Daivonex^{®} or Dovonex^{®} ointment by LEO Pharma) in which the calcipotriol is present in solution or a cream composition (marketed as Daivonex^{®} or Dovonex^{®} cream by LEO Pharma). The solvent in the ointment composition is propylene glycol which has the advantage of enhancing penetration of the active ingredient into the skin, leading to an improved efficacy, but which is also known to act as a skin irritant. Thus, it has been reported that the inclusion of propylene glycol in topical compositions frequently causes patients to develop contact dermatitis (one study reported a number of irritant reactions to propylene glycol of 12.5%, cf. M. Hannuksela et al., Contact Dermatitis 1, 1975, pp. 112-116), and the number of irritant reactions increases when propylene glycol is used in high concentrations (as reviewed by J. Catanzaro and J. Graham Smith, J. Am. Acad. Dermatol. 24, 1991, pp. 90-95). Due to the improved penetration of calcipotriol into the skin resulting, *inter alia,* from the presence of propylene glycol, Daivonex^{®} ointment has been found to be more efficacious in the treatment of psoriatic lesions than Daivonex^{®} cream, but has also caused skin irritation in a significant proportion of psoriasis patients.

It is therefore an object of the invention to provide a topical composition comprising a vitamin D derivative or analogue as the active ingredient, which has skin penetration and biological activity properties comparable to those of Daivonex^{®} ointment, but which does not contain propylene glycol as the solvent.

While vegetable oils and derivatives thereof such as medium-chain triglycerides based on coconut oil fractions have been used previously as alternatives to alcoholic solvents to formulate vitamin D derivatives in topical compositions, cf. US 2008/0239681, their power to dissolve vitamin D analogues such as calcipotriol is rather low. Thus, we have found that about 50% MCT is required to completely dissolve calcipotriol in a tested ointment composition. At this level of MCT, the composition is not physically stable in that pronounced phase separation is observed. An object of this invention was therefore to identify a mixture of non-alcoholic solvents adequate to provide complete dissolution of calcipotriol in an amount which is sufficiently low to substantially avoid phase separation when mixed with the ointment base. On the other hand, the mixture of solvents should be such that calcipotriol, which is known to be unstable in the presence of acidic substances or impurities, is not significantly degraded over the shelf-life of the ointment composition.

### SUMMARY OF THE INVENTION

Human skin, in particular the outer layer, the stratum corneum, provides an effective barrier against penetration of microbial pathogens and toxic chemicals. While this property of skin is generally beneficial, it complicates the dermal administration of pharmaceuticals in that a large quantity, if not most, of the active ingredient applied on the skin of a patient suffering from a dermal disease may not penetrate into the viable layers of the skin where it exerts its activity. To ensure an adequate penetration of the active ingredient to the dermis and epidermis, it is generally preferred to include the active ingredient in a dissolved state, typically in the presence of a solvent in the form of an alcohol, e.g. ethanol, or diol, e.g. propylene glycol. Propylene glycol is a well-known penetration enhancer, i.e. a substance which is capable of penetrating the stratum corneum and "draw" low-molecular components such as therapeutically active components in the vehicle into the epidermis. Propylene glycol may in itself give rise to significant skin irritation, and it is also capable of "drawing" low-molecular and potentially irritative components of the vehicle into the epidermis, leading to an overall irritative effect of conventional vehicles including propylene glycol. For this reason, the presence of propylene glycol as a solvent in compositions intended for the treatment of inflammatory skin diseases may exacerbate the inflammatory response.

In the research leading to the present invention, it was an object to identify a solvent which is equally effective to dissolve a sparingly soluble compound such as a vitamin D analogue as low-molecular alcohols or diols, but which is non-alcoholic. It has surprisingly been found that certain mixtures of solvents selected from different solvent classes have enabled the present inventors to provide a topical composition which, while ensuring satisfactory penetration of the vitamin D analogue in the viable layers of the skin, is homogeneous and physically stable, and in which the vitamin D analogue is chemically stable.

The invention relates to a storage stable, substantially anhydrous topical composition comprising a homogenous mixture of
(a) a therapeutically effective amount of calcipotriol in dissolved form;
(b) a solvent mixture consisting essentially of a fatty acid ester solvent selected from the group consisting of glyceryl esters, e.g. triglycerides of fatty acids, isopropyl esters of C₁₀₋₁₈ alkanoic or alkenoic acids, e.g. isopropyl myristate, isopropyl palmitate, isopropyl isostearate, isopropyl linoleate, isopropyl monooleate, or propylene glycol esters, e.g. propylene glycol dipelargonate, or mixtures thereof;
   a fatty alkyl ether co-solvent which is a compound of general formula I H(OCH₂C(R²)H)ₓR¹, wherein R¹ is straight or branched chain C₁₋₂₀ alkyl, each R² ls individually hydrogen or CH₃, and x is an integer of 2-60; and a lipophilic penetration enhancer selected from the group consisting of N-alkylpiperidone, N-alkylpyrrolidone, such as N-methylpyrrolidone or N-hydroxyalkylpyrrohidone, or dimethylacetamide, dimethylsulfoxide or 2-pyrrolidone;
   wherein the ratio of fatty acid ester solvent to fatty alkyl ether co-solvent to penetration enhancer is in the range of from 50:25:25 to 75:10:15;
   said solvent mixture being included in the composition in an amount which is sufficient to effectively dissolve said calcipotriol, and
(c) a substantially anhydrous pharmaceutically acceptable carrier.

In another aspect, the invention relates to a composition as disclosed herein for use in the treatment of dermatological diseases or conditions.

### BRIEF DESCRIPTION OF THE DRAWINGS

Fig. 1 is a graph showing the penetration of calcipotriol from a composition according to the invention.
Fig. 2 is a schematic representation of the activation of the gene encoding cathelicidin by vitamin D₃ in human keratinocytes. The mechanism of cathelicidin gene activation is used in a biological assay using reconstructed human epidermis (human keratinocytes cultured so as to form the epidermal layers characteristic of human skin) on which calcipotriol-containing compositions of the invention are applied to activate cathelicidin as described in detail in Example 6 below.

### DETAILED DISCLOSURE OF THE INVENTION

### Definitions

The term "vitamin D derivative" is intended to indicate a biologically active metabolite of vitamin D₃, such as calcitriol, or a precursor to such a metabolite, such as alfacalcidol.

The term "vitamin D analogue" is intended to indicate a synthetic compound comprising a vitamin D scaffold with sidechain modifications and/or modifications of the scaffold itself. The analogue exhibits a biological activity on the vitamin D receptor comparable to that of naturally occurring vitamin D compounds.

"Calcipotriol" is a vitamin D analogue of the formula

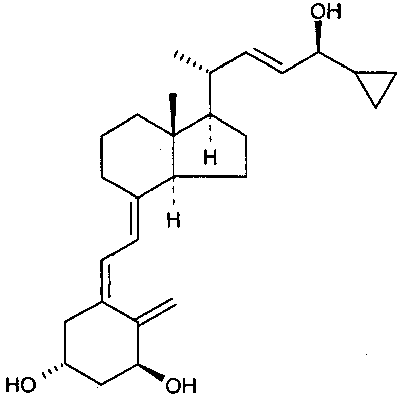

Calcipotriol has been found to exist in two crystalline forms, an anhydrate and a monohydrate. Calcipotriol monohydrate and its preparation are disclosed in WO 94/15912.

The term "storage stability" or "storage stable" is intended to indicate that the composition exhibits chemical and physical stability characteristics that permit storage of the composition for a sufficient period of time at refrigeration or, preferably, room temperature to make the composition commercially viable, such as at least 12 months, in particular at least 18 months, and preferably at least 2 years.

The term "chemical stability" or "chemically stable" is intended to mean "Chemical stability" indicates that no more than 10%, preferably no more than 5%, of the calcipotriol monohydrate degrades over the shelf-life of the product, typically 2 years, at room temperature. An approximation of chemical stability at room temperature is obtained by subjecting the composition to accelerated stability studies at 40°C. If less than about 10% of the substance has degraded after 3 months at 40°C, this is usually taken to correspond to a shelf-life of 2 years at room temperature. When the active ingredient included in the composition, "chemical stability" usually indicates that the calcipotriol does not degrade significantly over time to 24-epi calcipotriol or other degradation products of calcipotriol in the finished pharmaceutical product.

The term "physical stability" or "physically stable" is intended to mean that the composition retains its macroscopic and microscopic appearance over the shelf-life of the product, e.g. that the calcipotriol does not precipitate from the solvent phase or that there is no visible phase separation of the solvent phase and the carrier phase.

The term "substantially anhydrous" is intended to mean that the content of free water in the ointment composition does not exceed about 2% by weight, preferably not about 1% by weight, of the composition.

The term "medium-chain triglycerides" is used to indicate triglyceride esters of fatty acids with a chain length of 6-12 carbon atoms. A currently favoured example of such medium chain triglycerides is a mixture of caprylic (C₈) and capric (C₁₀) triglycerides, e.g. available under the trade name Miglyol 812.

The term "solubilization capacity" is intended to indicate the ability of a solvent or mixture of solvents to dissolve a given substance, expressed as the amount required to effect complete dissolution of the substance.

The term "skin penetration" is intended to mean the diffusion of the active ingredient into the different layers of the skin, i.e. the stratum corneum, epidermis and dermis.

The term "skin permeation" is intended to mean the flux of the active ingredient through the skin into the systemic circulation or, in case of *in vitro* studies such as those reported in Example 5 below, the receptor fluid of the Franz cell apparatus used in the experiment.

The term "biological activity" is intended to mean the activity of a vitamin D derivative or analogue when applied to skin in a composition of the invention. The biological activity of compositions is determined in an *in vitro* assay measuring the activation of a target gene encoding cathelicidin in a reconstructed human epidermis model involving cultured human keratinocytes, as described in detail in Example 6 below.

### Embodiments of the invention

The vitamin D derivative or analogue included in the present composition may be selected from calcipotriol, calcitriol, tacalcitol, maxacalcitol, paricalcitol and alfacalcidol. A preferred vitamin D analogue which has been shown to be effective in the treatment of psoriasis is calcipotriol. Before dissolution in the solvent mixture, calcipotriol may be in the form of anhydrate or monohydrate, preferably the monohydrate.

In the solvent mixture used in the present composition, the fatty acid ester solvent component is preferably selected from glyceryl esters, e.g. triglycerides of fatty acids, such as C₆₋₂₄ fatty acids, while isopropyl esters of C₁₀₋₁₈ alkanoic or alkenoic acids such as isopropyl myristate, isopropyl palmitate, isopropyl isostearate, isopropyl linoleate, isopropyl monooleate, as well as octyldodecanol, or propylene glycol esters, e.g. propylene glycol dipelargonate, may also be employed. While medium chain triglycerides (as defined herein) are generally preferred, they cannot be used on their own as a large quantity (about 50% by weight of the composition) is require to effect complete dissolution of the vitamin D analogue. The inclusion of this high amount of fatty acid ester solvent in the ointment base has been found to result in physical instability (phase separation) and also, when the vitamin D analogue is calcipotriol, chemical instability due, most likely, to the presence of acidic impurities in the form of fatty acid residues in the solvent.

The solvent mixture further comprises a fatty alkyl ether co-solvent which is preferably a compound of general formula I, H(OCH₂C(R²)H)ₓR¹, wherein R¹ is straight or branched chain C₁₋₂₀ alkyl, each R² is individually hydrogen or CH₃, and x is an integer of 2-60. Examples of suitable co-solvents are polyoxypropylene-15-stearyl ether, polyoxypropylene-11-stearyl ether, polyoxypropylene-14-butyl ether, polyoxypropylene-10-cetyl ether, polyoxypropylene-3-myristyl ether, polyoxypropylene-5-ceteth 20. Apart from increasing the solubilization capacity of the mixture, it has surprisingly been found that including this type of co-solvent increases the chemical stability of calcipotriol included in the composition.

The solvent mixture further comprises a penetration enhancer selected from the group consisting of N-alkylpiperidone, N-alkylpyrrolidone, such as N-methylpyrrolidone, N-hydroxyalkylpyrrolidone, dimethylacetamide or dimethylsulfoxide. The penetration enhancer is preferably N-methylpyrrolidone or 2-pyrrolidone.

It has surprisingly been found that in the present mixture of solvents each solvent acts to increase the solubilization capacity of the mixture such that a far smaller amount of each solvent in the mixture is needed to dissolve all of the calcipotriol than of each of the solvents alone. Thus, the amount of solvent mixture included in the composition is preferably in the range of about 0.1-15% by weight, in particular about 1-14% by weight, about 2-12% by weight, or about 5-10% by weight, such as about 10% by weight, of the composition. It has been found that this amount of solution may be homogenously incorporated in the composition without any phase separation or precipitation of the vitamin D derivative or analogue being observed.

In the present composition the ratio of fatty acid ester solvent to fatty alkyl ether co-solvent to penetration enhancer is favourably in the range of from about 40:25:35 to about 99:0.9:0.1. A preferred ratio of fatty acid ester solvent to fatty alkyl ether co-solvent to penetration enhancer is in the range of from about 50:25:25 to about 75:10:15. A ratio of fatty acid ester solvent to fatty alkyl ether co-solvent to penetration enhancer of about 60:15:25 has been found to provide a favourable balance between solubilization capacity, skin penetration, physical stability and chemical stability of the active ingredient.

The present composition preferably comprises about 0.5-12% w/w, about 1-10% w/w, about 2-8% w/w or about 4-7% w/w or about 5% w/w or about 6% w/w of the fatty acid ester solvent.

The present composition preferably comprises about 0.1-5% w/w, about 0.2-4% w/w, about 1-3 % w/w or about 1.2-2% w/w such as about 1.5% w/w of the fatty alkyl ether co-solvent.

The present composition preferably comprises about 0.1-6% w/w, about 0.3-5% w/w, about 0.5-4% w/w, or about 1-3.5% w/w, or about 2-3% w/w or about 2.5% w/w of the penetration enhancer.

The ointment carrier may be a hydrocarbon or mixture of hydrocarbons with chain lengths ranging from C₅ to C₆₀. A frequently used ointment carrier is petrolatum, or white soft paraffin, which is composed of hydrocarbons of different chain lengths peaking at about C₄₀₋₄₄, or a mixture of petrolatum and liquid paraffin (consisting of hydrocarbons of different chain lengths peaking at C₂₈₋₄₀). While petrolatum provides occlusion of the treated skin surface, reducing transdermal loss of water and potentiating the therapeutic effect of the active ingredient in the composition, it tends to have a greasy or tacky feel which persists for quite some time after apptication, and it is not easily spreadable. It may therefore be preferred to employ paraffins consisting of hydrocarbons of a somewhat lower chain length, such as paraffins consisting of hydrocarbons with chain lengths peaking at C₁₄₋₁₆, C₁₈₋₂₂, C₂₀₋₂₂, C₂₀₋₂₆ or mixtures thereof. It has been found that such paraffins are more cosmetically acceptable in that they are less greasy or tacky on application and more easily spreadable. They are therefore expected to result in improved patient compliance. Suitable paraffins of this type, termed petrolatum jelly, are manufactured by Sonneborn and marketed under the trade name Sonnecone, e.g. Sonnecone CM, Sonnecone DM1, Sonnecone DM2 and Sonnecone HV. These paraffins are further disclosed and characterized in WO 2008/141078. In addition to their favourable cosmetic properties, it has surprisingly been found that compositions containing these paraffins as carriers are more tolerable than compositions containing conventional paraffins. (The hydrocarbon composition of the paraffins has been determined by gas chromatography).

To impart a desired viscosity to the present composition, it may suitably include a lipophilic viscosity-increasing ingredient such as a wax. The wax may be a mineral wax composed of a mixture of high molecular weight hydrocarbons, e.g. saturated C₃₅₋₇₀ alkanes, such as microcrystalline wax. Alternatively, the wax may be a vegetable or animal wax, e.g. esters of C₁₄₋₃₂ fatty acids and C₁₄₋₃₂ fatty alcohols, such as beeswax. The amount of viscosity-increasing ingredient may vary according to the viscosifying power of the ingredient, but may typically be in the range of about 1-20% by weight of the composition. When the viscosity-increasing ingredient is microcrystalline wax it is typically present in an amount in the range of about 5-15% by weight, e.g. about 10% by weight, of the composition.

The composition may additionally comprise an emollient which may act to soften the thickened epidermis of the psoriatic plaques. A suitable emollient for inclusion in the present composition may be a silicone wax or a volatile silicone oil as the presence of silicone has additionally been found to aid penetration of calcipotriol into the skin. Compositions including a silicone have also been found to result in less skin irritation. Suitable silicone oils for inclusion in the present composition may be selected from cyclomethicone and dimethicone. The amount of silicone oil included in the present composition is typically in the range of 1-10% w/w, such as about 5% w/w.

Calcipotriol is known to be a substance which is extremely sensitive to acid conditions (pH below about 7.0 in aqueous compositions or acidic reacting substances in nonaqueous compositions) which contribute to the rapid degradation of calcipotriol. To ensure an adequate chemical stability of the substance throughout the shelf-life of the composition, it may be advisable to include a compound capable of neutralizing acidic impurities which may be present in one or more of the excipients of the composition and which are detrimental to the chemical stability of calcipotriol. In an anhydrous composition, the acid neutralizing compound may advantageously be a lipophilic compound such as an amine such as triethanolamine, trometamol, monoethanol amine or diethanolamine, included in the composition in an amount of about 0.1-2% w/w.

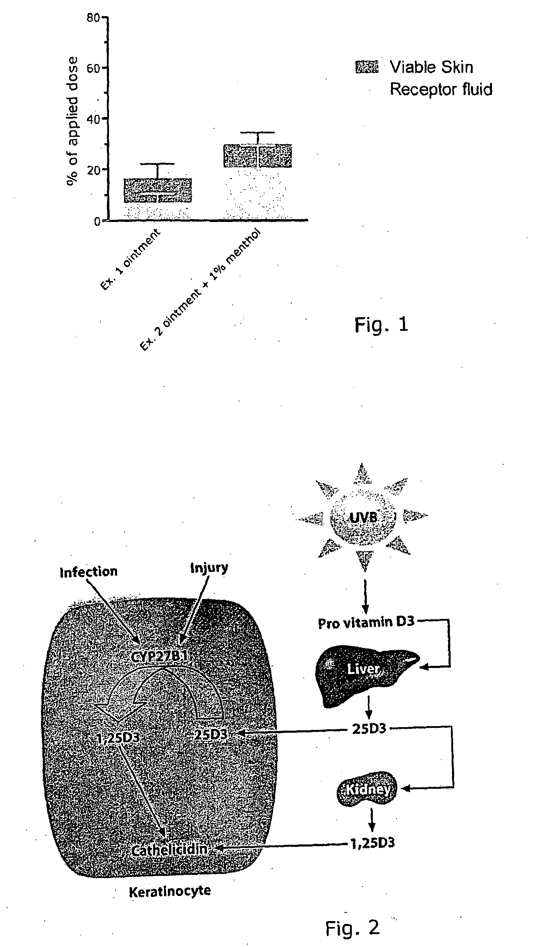

The present composition may also comprise other components commonly used in dermal formulations, e.g. antioxidants (e.g. alpha-tocopherol), preservatives, pigments, skin soothing agents, skin healing agents and skin conditioning agents such as urea, glycerol, allantoin or bisabolol, cf. CTFA Cosmetic Ingredients Handbook, 2nd Ed., 1992. In a favoured embodiment, the composition may comprise an anti-irritative agent such as menthol, eucalyptol or nicotinamide. A currently preferred anti-irritative agent is menthol as it has been found also to increase the penetration of calcipotriol into the skin, cf. Fig. 1. The menthol may be included in the composition in an amount of about 0.05-0.1% w/w, in particular about 0.08% w/w, of the composition.

In one embodiment, the composition comprises
0.003-0.008% w/w calcipotriol (as monohydrate)
5-8% w/w medium chain triglycerides
1-3% w/w N-methylpyrrolidone
1-2% w/w polyoxypropylene-15-stearyl ether
80-90% w/w paraffin carrier.

The composition of the invention may be used in the treatment of psoriasis, sebopsoriasis, pustulosis palmoplantaris, dermatitis, ichtyosis, rosacea and acne and related skin diseases by topically administering an effective amount of a composition according to the invention to a patient in need of such treatment. Said method preferably comprises topical administration once or twice a day of a therapeutically sufficient dosage of said composition. To that end, the composition according to the invention preferably contains about 0.001-0.5 mg/g, preferably about 0.002-0.25 mg/g, in particular 0.005-0.05 mg/g, of the vitamin D derivative or analogue. It is envisaged that the present composition may advantageously be used for maintenance treatment of these dermal diseases, i.e. continued treatment after the disappearance of visible symptoms of the disease in order to delay recurrence of the symptoms.

To provide a more effective treatment of the dermal disease or conditions, it may be desirable to include one or more additional therapeutically active ingredients in the composition. Examples of such additional active ingredients include, but are not limited to, anti-inflammatory drugs such as corticosteroids, such as betamethasone and esters thereof, e.g. the valerate or dipropionate ester, clobetasol or esters thereof, such as the propionate, hydrocortisone or esters thereof, such as the acetate; non-steroidal anti-inflammatory drugs such as naproxen, indomethacin, diclofenac, ibuprofen, dexibuprofen, ketoprofen, flurbiprofen, piroxicam, tenoxicam, lornoxicam or nabumeton, phosphodiesterase 4 inhibitors (e.g. compounds disclosed in WO 2008/077404, WO 2008/104175, WO 2008/128538 or WO 2010/069322) or p38 MAP kinase inhibitors (e.g. compounds disclosed in WO 2005/009940 or WO 2006/063585).

It has surprisingly been found that the solvent mixture in the present composition is capable of dissolving certain types of plastic used for the container (e.g. a tube) or as an inner coating of the container used to store the composition before use. The material used to store the composition should therefore be carefully selected such that dissolution or other forms of degradation may be minimized or avoided altogether.

The invention is further illustrated by the following examples which are not in any way intended to limit the scope of the invention as claimed.

### EXAMPLES

### Example 1

### Testing different solvent mixtures

The solubility of calcipotriol monohydrate in the compositions shown in Table 1 below was tested.

**Table 1**

| Ingredient mg/g | sol. 1 | sol. 2 | sol. 3 | sol. 4 | sol. 5 | sol. 6 | sol. 7 | sol. 8 | sol. 9 |
|---|---|---|---|---|---|---|---|---|---|
| Miglyol 812 | 1.2 | | 1.2 | 60 | | 60 | 1.2 | | 1.2 |
| NMP | 0.5 | 0.5 | 0.5 | 25 | 25 | 25 | | | |
| 2-pyrrolidone | | | | | | | 0.5 | 0.5 | 0.5 |
| Arlamol E | | 0.3 | 0.3 | | 15 | 15 | | 0.3 | 0.3 |
| Paraffin light liquid | 998.3 | 999.2 | 998 | 915 | 960 | 900 | 998.3 | 999.2 | 998 |

The solubility was determined by shaking 3 ml of the vehicles with an excess of calcipotriol monohydrate for 24 hours at 25±2°C in a temperature-controlled cabin. The experiments were performed as double determinations. After 24 hours the suspensions were filtered through a Millex^{®}-LCR filter, and the filtrate was transferred to a clean reaction tube and diluted with isopropanol. The concentration was determined by reversed phase HPLC with UV detection (264 nm) against a standard curve.

The solubility of calcipotriol monohydrate in the respective solutions is shown in Table 2 below.

**Table 2**

| | |
|---|---|
| solution 1 | ~ 1 µg/g |
| solution 2 | ~ 1 µg/g |
| solution 3 | ~ 1 µg/g |
| solution 4 | ~ 97 µg/g |
| solution 5 | ~ 60 µg/g |
| solution 6 | ~ 130 µg/g |
| solution 7 | ~ 1 µg/g |
| solution 8 | ~ 1 µg/g |
| solution 9 | ~ 1 µg/g |

It appears from the results of this experiment that the solubilizing capacity of solution 6, containing medium chain triglycerides, N-methylpyrrolidone and polyoxypropylene-15-stearyl ether is significantly superior to that of the other solutions.

### Example 3

### Stability of calcipotriol in different solvent mixtures

The composition of three solvent mixtures containing 25% NMP and 0% or 15% polyoxypropylene-15-stearyl ether is shown in Table 3 below.

**Table 3**

| Ingredient (mg/g) | solvent mix 1 | solvent mix 2 | solvent mix 3 |
|---|---|---|---|
| Calcipotriol monohydrate | 0.052 | 0.052 | 0.052 |
| Medium chain triglycerides | 750 | 732 | 600 |
| N-methylpyrrolidone | 250 | 250 | 250 |
| DMA | | 18 | |
| Arlamol^{®} E | | | 15 |

The solvent mixtures were stored for 1 month at 40°C and for 3 months at 25°C and 40°C after which the content of calcipotriol was determined by HPLC. The results are shown in Table 4 below as the percentage of the initial determination.

**Table 4**

| Storage | mix 1 % of start | mix 2 % of start | mix 3 % of start |
|---|---|---|---|
| Start | 100.0 | 100.0 | 100.0 |
| 3 months/25°C | 70.5 | nd | 98.1 |
| 1 month/40°C | 71.8 | 68.1 | 99.6 |
| 3 months/40°C | 26.8 . | 23.0 | 99.1 |

It appears from the results that the only solvent mixture showing a satisfactory stability is solvent mixture 3 containing 15% Arlamol^{®} E (polyoxypropylene-15-stearyl ether).

### Example 4

### Preparation of ointment compositions containing calcipotriol

A solution of calcipotriol monohydrate in N-methylpyrrolidone was mixed with medium chain triglycerides and polyoxypropylene-15-stearyl ether.

To prepare Composition A, Sonnecone DM1 and microcrystalline wax was melted at 80-85°C, and a solution of DL-a-tocopherol in liquid paraffin was heated at 80°C with stirring until melting and triethanolamine was added. After cooling to 70-75°C, the solvent mixture containing calcipotriol was added with stirring. After cooling to about 40°C, menthol was added and the resulting ointment was stirred with cooling to below 30°C. The ointment was filled into 15 g tubes and stored before use.

To prepare Composition B, white soft paraffin was melted at 80-85°C and cooled to 70-75°C, and the solvent mixture was added with stirring. The resulting ointment was stirred with cooling to below 30°C and filled into 15 g tubes for storing before use.

A solution of calcipotriol monohydrate in 2-pyrrolidone was mixed with medium chain triglycerides and polyoxypropylene-15-stearyl ether.

To prepare Composition C, Sonnecone DM1 and microcrystalline wax was melted at 80-85°C, and a solution of DL-α-tocopherol in liquid paraffin was heated at 80°C with stirring until melting and triethanolamine was added. After cooling to 70-75°C, the solvent mixture containing calcipotriol was added with stirring. After cooling to about 40°C, menthol was added and the resulting ointment was stirred with cooling to below 30°C. The ointment was filled into 15 g tubes and stored before use.

To prepare Composition D, white soft paraffin was melted at 80-85°C and cooled to 70-75°C, and the solvent mixture was added with stirring. The resulting ointment was stirred with cooling to below 30°C and filled into 15 g tubes for storing before use.

**Composition**

| Ingredient | Comp. A | Comp. B | Comp. C | Comp. D |
|---|---|---|---|---|
| Calcipotriol monohydrate | 50 µg | 50 µg | 50 µg | 50 µg |
| Medium chain triglycerides | 60 mg | 60 mg | 60 mg | 60 mg |
| N-methylpyrrolidone | 25 mg | 25 mg | | |
| 2-pyrrolidone | | | 25 mg | 25 mg |
| Polyoxypropylene-15-stearyl ether | 15 mg | 15 mg | 15 mg | 15 mg |
| Menthol | 0.8 mg | | 0.8 mg | |
| Triethanolamine | 10 mg | 10 mg | 10 mg | 10 mg |
| Paraffin, liquid | 50 mg | | 50 mg | |
| DL-α-tocopherol | 0.02 mg | | 0.02 mg | |
| Petrolatum jelly white (Sonnecone DM1) | 739.2 mg | | 739.2 mg | |
| Microcrystalline wax (Multiwax 180 MH) | 100 mg | | 100 mg | |
| White soft paraffin | | 890 mg | | 890 mg |

### Example 5

### Penetration studies

To investigate the skin penetration and permeation of calcipotriol from compositions of the invention, a skin diffusion experiment was conducted. Full thickness skin from pig ears was used in the study. The ears were kept frozen at -18°C before use. On the day prior to the experiment the ears were placed in a refrigerator (5±3°C) for slow defrosting. On the day of the experiment, the hairs were removed using a veterinary hair trimmer. The skin was cleaned for subcutaneous fat using a scalpel and two pieces of skin were cut from each ear and mounted on Franz diffusion cells in a balanced order.

Static Franz-type diffusion cells with an available diffusion area of 3.14 cm² and receptor volumes ranging from 8.6 to 11.1 ml were used in substantially the manner described by T.J. Franz, "The finite dose technique as a valid in vitro model for the study of percutaneous absorption in man", in Current Problems in Dermatology, 1978, J.W.H. Mall (Ed.), Karger, Basel, pp. 58-68. The specific volume was measured and registered for each cell. A magnetic bar was placed in the receptor compartment of each cell. After mounting the skin, physiological saline (35°C) was filled into each receptor chamber for hydration of the skin. The cells were placed in a thermally controlled water bath which was placed on a magnetic stirrer set at 400 rpm. The circulating water in the water baths was kept at 35±1°C resulting in a temperature of about 32°C on the skin surface. After one hour the saline was replaced by receptor medium, 0.04 M isotonic phosphate buffer, pH 7.4 (35°C), containing 4% bovine serum albumin. Sink conditions were maintained at all times during the period of the study, i.e. the concentration of the active compounds in the receptor medium was below 10% of the solubility of the compounds in the medium.

The *in vitro* skin permeation of each test composition was tested in 6 replicates (i.e. n=6). Each test composition was applied to the skin membrane at 0 hours in an intended dose of 4 mg/cm². A glass spatula was used for the application, and the residual amount of the composition was determined so as to give the amount of the composition actually applied on the skin.

The skin penetration experiment was allowed to proceed for 21 hours. Samples were then collected from the following compartments:

The stratum corneum was collected by tape stripping 10 times using D-Squame^{®} tape (diameter 22 mm, CuDerm Corp., Dallas, Texas, USA). Each tape strip is applied to the test area using a standard pressure for 5 seconds and removed from the test area in one gentle, continuous move. For each repeated strop, the direction of tearing off was varied. The viable epidermis and dermis was then sampled from the skin in a similar fashion.

Samples (1 ml) of the receptor fluid remaining in the diffusion cell were collected and analysed.

The concentration of calcipotriol in the samples were determined by LC mass spectrometry.

The results appear from Figure 1 below which shows the amount of calcipotriol found in viable skin (dermis and epidermis) and receptor fluid in % of the applied dose. The results show that the addition of menthol to the composition leads to a significant increase in skin permeation of calcipotriol.

### Example 6

### Biological activity of the compositions

As shown in figure 2 below, cathelicidin is an antimicrobial peptide expressed in human keratinocytes. The expression of cathelicidin is strongly induced on infection of the skin or disruption of the skin barrier. In psoriasis, the level of cathelicidin is increased in lesional skin of psoriasis patients. It has been found that the expression of the gene encoding cathelicidin may be induced by vitamin D₃ or vitamin D analogues such as calcipotriol (cf. TT Wang et al, J. Immunol. 173(5), 2004, pp. 2909-2912; J Schauber et al., Immunology 118(4), 2006, pp. 509-519; Schauber and Gallo, J. Allergy Clin Immunol 122, 2008, pp. 261-266; M. Peric et al., PloS One 4(7), July 22, 2009, e6340) through binding to the vitamin D receptor. This finding has been utilized to develop an assay in which the uptake and biological activity of calcipotriol in human keratinocytes from the tested compositions has been determined by measuring the level of induction of the gene encoding cathelicidin.

In the assay, composition A prepared as descibed in Example 2 above was applied topically in triplicate on reconstructed human epidermis consisting of normal human keratinocytes cultured for 12 days on 0.5 cm² polycarbonate filters (available from SkinEthic^{®} Laboratories, Nice, France) in an amount of 10 µl. The tissue was treated for two days followed by separation of the epidermis from the polycarbonate filter and snap-frozen in liquid nitrogen. RNA was extracted from the cells and cDNA synthesized by conventional procedures. Quantitative real-time PCR (qPCR) was then performed using the following assays from Applied Biosystems: CAMP Hs0018038_m1 and GAPDH Hs99999905_m1. The expression levels of cathelicidin were normalized to GAPDH and a relative quantification was made by comparison with Daivonex^{®} ointment.

The results show a 4.4 fold increase in the biological activation of cathelicidin relative to that obtained with Daivonex^{®} ointment.

### Example 7

### Local tolerance study in minipigs

The local tolerability of Composition A of Example 2 was assessed when administered daily by dermal application to minipigs for 4 weeks. Each day the animals were exposed to the test items for 8 hours.

The study was conducted in 10 female Göttingen SPF minipigs. Each animal had 6 application sites and received a volume of 250 mg test formulation per application site. Clinical signs were recorded daily and skin reactions at the application sites were scored once daily prior to start of dosing and, furthermore, on the day of necropsy in relation to erythema and oedema. Food consumption was recorded daily and the body weight weekly. At the end of the treatment period a gross necropsy was performed on all animals and skin samples were collected from histopathological examination.

The results show no adverse tretment-related clinical signs were observed during the study. No scores in relation to erythema were observed for Composition A of Example 2. The results imply that compositions of the invention will be well tolerated in human patients as well.

## Claims

1. A storage stable, substantially anhydrous topical composition comprising a homogenous mixture of
(a) a therapeutically effective amount of calcipotriol in dissolved form;
(b) a solvent mixture consisting essentially of a fatty acid ester solvent selected from the group consisting of glyceryl esters, e.g. triglycerides of fatty acids, isopropyl esters of C₁₀₋₁₈ alkanoic or alkenoic acids, e.g. isopropyl myristate, isopropyl palmitate, isopropyl isostearate, isopropyl linoleate, isopropyl monooleate, or propylene glycol esters, e.g. propylene glycol dipelargonate, or mixtures thereof;
a fatty alkyl ether co-solvent which is a compound of general formula I H(OCH₂C(R²)H)ₓR¹, wherein R¹ is straight or branched chain C₁₋₂₀ alkyl, each R² is individually hydrogen or CH₃, and x is an integer of 2-60; and
a lipophilic penetration enhancer selected from the group consisting of N-alkylpiperidone, N-alkylpyrrolidone, such as N-methylpyrrolidone or N-hydroxyalkylpyrrolidone, or dimethylacetamide, dimethylsulfoxide or 2-pyrrolidone;
wherein the ratio of fatty acid ester solvent to fatty alkyl ether co-solvent to penetration enhancer is in the range of from 50:25:25 to 75:10:15;
said solvent mixture being included in the composition in an amount which is sufficient to effectively dissolve said calcipotriol, and
(c) a substantially anhydrous pharmaceutically acceptable carrier.

2. A composition according to claim 1, wherein the fatty acid ester solvent consists essentially of medium chain triglycerides.

3. A composition according to claim 1 or 2, wherein the fatty alkyl ether co-solvent is polyoxypropylene-15-stearyl ether, polyoxypropylene-11-stearyl ether, polyoxypropylene-14-butyl ether, polyoxypropylene-10-cetyl ether, polyoxypropylene-3-myristyl ether, polyoxypropylene-5-ceteth 20.

4. A composition according to any one of claims 1-3, wherein said solvent mixture (b) is included in the composition in an amount in the range of from 0.1% to 15% by weight of the composition, preferably 1-14% by weight, 2-12% by weight or 5-10% by weight, such as 10% by weight of the composition.

5. A composition according to any one of claims 1-4, wherein the ratio of fatty acid ester solvent to fatty elkyl ether co-solvent to penetration enhancer is 60:15:25.

6. A composition according to any one of claims 1-5 comprising 0.5-12% w/w, 1-10% w/w, 2-8% w/w, 4-7 or 5% w/w or 6% w/w of the fatty acid ester solvent.

7. A composition according to any one of claims 1-6 comprising 0.1-5% w/w, 0.2-4% w/w, 1-3% w/w or 1.2-2% w/w such as 1.5% w/w of the fatty alkyl ether co-solvent.

8. A composition according to any one of claims 1-7 comprising 0.1-6% w/w, 0.3-5% w/w, 0.5-4% w/w, or 1-3.5% w/w, 2-3% w/w, or 2.5% w/w of the penetration enhancer.

9. A composition according to any one of claims 1-8 further comprising an anti-irritative agent.

10. A composition according to claim 9, wherein the anti-irritative agent is menthol, eucalyptol or nicotinamide.

11. A composition according to any one of claims 1-10 further comprising an agent capable of neutralizing acidic impurities or degradation products present in the composition.

12. A composition according to claim 11, wherein said agent is an amine such as triethanolamine, trometamol, monoethanolamine or diethanolamine.

13. A composition according to any one of claims 1-12, wherein the carrier comprises at least one paraffin selected from paraffins consisting of hydrocarbons with chain lengths from C₅ to C₆₀, the chain lengths peaking at C₁₄₋₁₆, C₁₈₋₂₂, C₂₀₋₂₂, C₂₀₋₂₆, C₂₈₋₄₀, and C₄₀₋₄₄ (as determined by gas chromatography), or mixtures thereof.

14. A composition according to any one of claims 1-13, further comprising a viscosity-increasing ingredient.

15. A composition according to claim 14, wherein the viscosity-increasing ingredient is a wax.

16. A composition according to any one of claims 1-15, further comprising a silicone wax or a volatile silicone oil.

17. A composition according to claim 16, wherein the volatile silicone oil is cyclomethicone or dimethicone.

18. A composition according to any one of claims 1-17 comprising 0.001-0.5 mg/g, preferably 0.002-0.25 mg/g, in particular 0.005-0.05 mg/g, of calcipotriol.

19. A composition according to any one of claims 1-18 comprising
0.003-0.008% w/w calcipotriol
5-8% w/w medium chain triglycerides
1-3% w/w N-methylpyrrolidone
1-2% w/w polyoxypropylene-15-stearyl ether
80-90% w/w paraffin carrier.

20. A composition according to any one of claims 1-19, further comprising one or more additional therapeutically active ingredients.

21. A composition according to claim 20, wherein such additional active ingredients are selected from the group consisting of anti-inflammatory drugs such as corticosteroids, such as betamethasone and esters thereof, e.g. the valerate or dipropionate ester, clobetasol or esters thereof, such as the propionate, hydrocortisone or esters thereof, such as the acetate; non-steroidal anti-inflammatory drugs such as naproxen, indomethacin, diclofenac, ibuprofen, dexibuprofen, ketoprofen, flurbiprofen, piroxicam, tenoxicam, lornoxicam or nabumeton, phosphodiesterase 4 inhibitors or p38 MAP kinase inhibitors.

22. A composition according to claim 21, wherein the additional active ingredient is selected from the group consisting of betamethasone and an ester thereof, e.g. the valerate or dipropionate ester.

23. A composition according to any of claims 1-22 for use in the treatment of dermatological diseases or conditions.

24. A composition according to claim 23 for use in the treatment of dermatological diseases or conditions selected from the group consisting of psoriasis, pustulosis palmoplantaris, ichtyosis, rosacea, dermatitis and acne.

## Patentansprüche

1. Lagerstabile, im Wesentlichen wasserfreie topische Zusammensetzung, umfassend ein homogenes Gemisch von
(a) einer therapeutisch wirksamen Menge von Calcipotriol in gelöster Form;
(b) einem Lösungsmittelgemisch, bestehend im Wesentlichen aus einem Fettsäureester-Lösungsmittel, das ausgewählt ist unter Glycerinestern, z. B. Triglyceriden von Fettsäuren, Isopropylestern von C₁₀₋₁₈-Alkan- oder Alkensäuren, z.B. Isopropylmyristat, Isopropylpalmitat, Isopropylisostearat, Isopropyllinoleat, Isopropylmonooleat, oder Propylenglykolestern, z. B. Propylenglycoldipelargonat, oder Gemischen davon;
einem Fettalkylether-Co-Lösungsmittel, das eine Verbindung der allgemeinen Formel I H(OCH₂C(R²)H)ₓR¹ ist, wobei R¹ eine lineare oder verzweigte C₁₋₂₀-Alkylkette ist, jedes R² unabhängig voneinander Wasserstoff oder CH₃ ist und x eine ganze Zahl von 2-60 ist; und
einem lipophilen Penetrationsverstärker, der ausgewählt ist unter N-Alkylpiperidon, N-Alkylpyrrolidon, wie N-Methylpyrrolidon, oder N-Hydroxyalkylpyrrolidon, oder Dimethylacetamid, Dimethylsulfoxid oder 2-Pyrrolidon,
wobei das Verhältnis von Fettsäureester-Lösungsmittel zu Fettalkylether-Co-Lösungsmittel zu Penetrationsverstärker im Bereich von 50:25:25 bis 75:10:15 liegt und das Lösungsmittelgemisch in der Zusammensetzung in einer Menge enthalten ist, die ausreichend ist, um Calcipotriol effektiv zu lösen, und
(c) einem im Wesentlichen wasserfreien pharmazeutisch verträglichen Träger.

2. Zusammensetzung nach Anspruch 1, wobei das Fettsäureester-Lösungsmittel im wesentlichen aus mittelkettigen Triglyceriden besteht.

3. Zusammensetzung nach Anspruch 1 oder 2, wobei das Fettalkylether-Co-Lösungsmittel Polyoxypropylen-15-stearylether, Polyoxypropylen-11-stearylether, Polyoxypropylen-14-Butylether, Polyoxypropylen-10-cetylether, Polyoxypropylen-3-myristylether, oder Polyoxypropylen-5-Ceteth 20 ist.

4. Zusammensetzung nach einem der Ansprüche 1-3, wobei das Lösungsmittelgemisch (B) in der Zusammensetzung in einer Menge im Bereich von 0,1 % bis 15% des Gewichts der Zusammensetzung, vorzugsweise 1-14 Gew.-%, 2-12 Gew.-% oder 5-10 Gew.-%, wie 10 Gew.-%, des Gewichts der Zusammensetzung enthalten ist.

5. Zusammensetzung nach einem der Ansprüche 1-4, wobei das Verhältnis von Fettsäureester-Lösungsmittel zu Fettalkylether-Co-Lösungsmittel zu Penetrationsverstärker 60:15:25 ist.

6. Zusammensetzung nach einem der Ansprüche 1-5, umfassend 0,5-12 Gew.-%, 1-10 Gew.-%, 2-8 Gew.-%, 4-7 oder 5 Gew.-%, oder 6 Gew.-% des Fettsäureester-Lösungsmittels.

7. Zusammensetzung nach einem der Ansprüche 1-6, umfassend 0,1-5 Gew.-%, 0,2-4 Gew.-%, 1-3 Gew.-% oder 1,2-2 Gew.-%, wie 1,5 Gew.-% des Fettalkylether-Co-Lösungsmittels.

8. Zusammensetzung nach einem der Ansprüche 1-7, umfassend 0,1-6 Gew.-%, 0,3-5 Gew.-%, 0,5-4 Gew.-%, oder 1-3,5 Gew.-%, 2-3 Gew.-% oder 2,5 Gew.-% des Penetrationsverstärkers.

9. Zusammensetzung nach einem der Ansprüche 1-8, weiterhin umfassend ein antiirritatives Mittel.

10. Zusammensetzung nach Anspruch 9, wobei das anti-irritative Mittel Menthol, Eucalyptol oder Nicotinsäureamid ist.

11. Zusammensetzung nach einem der Ansprüche 1-10, weiterhin umfassend ein Mittel zur Neutralisierung saurer Verunreinigungen oder Abbauprodukte, die in der Zusammensetzung enthalten sind.

12. Zusammensetzung nach Anspruch 11, wobei das Mittel ein Amin ist, wie Triethanolamin, Trometamol, Monoethanolamin oder Diethanolamin.

13. Zusammensetzung nach einem der Ansprüche 1-12, wobei der Träger mindestens ein Paraffin umfasst, ausgewählt aus Paraffinen, welche aus Kohlenwasserstoffen mit einer Kettenlänge von C₅ bis C₆₀ bestehen, deren Kettenlängenverteilung (mittels Gaschromatographie bestimmte) Maxima bei C₁₄-₁₆, C₁₈₋₂₂, C₂₀₋₂₂, C₂₀₋₂₆, C₂₈₋₄₀ und C₄₀₋₄₄ besitzt, oder Gemische davon.

14. Zusammensetzung nach einem der Ansprüche 1-13, weiterhin umfassend einen die Viskosität erhöhenden Bestandteil.

15. Zusammensetzung nach Anspruch 14, wobei der die Viskosität erhöhende Bestandteil ein Wachs ist.

16. Zusammensetzung nach einem der Ansprüche 1-15, weiterhin umfassend ein Silikonwachs oder ein flüchtiges Silikonöl.

17. Zusammensetzung nach Anspruch 16, wobei das flüchtige Silikonöl Cyclomethicon oder Dimethicon ist.

18. Zusammensetzung nach einem der Ansprüche 1-17, umfassend 0,001 bis 0,5 mg/g, vorzugsweise 0,002 bis 0,25 mg/g, insbesondere 0,005 bis 0,05 mg/g Calcipotriol.

19. Zusammensetzung nach einem der Ansprüche 1-18, umfassend
0,003 bis 0,008 Gew.-% Calcipotriol
5-8 Gew.-% mittelkettige Triglyceride
1-3 Gew.-% N-Methylpyrrolidon
1-2 Gew.-% Polyoxypropylen-15-stearylether
80-90 Gew.-% Paraffinträger.

20. Zusammensetzung nach einem der Ansprüche 1-19, weiterhin umfassend einen oder mehrere zusätzliche therapeutische Wirkstoffe.

21. Zusammensetzung nach Anspruch 20, wobei solche zusätzlichen Wirkstoffe ausgewählt sind unter entzündungshemmenden Arzneimitteln, wie Corticosteroiden, etwa Betamethason und deren Ester, z. B. dem Valerat- oder Dipropionatester, Clobetasol oder Estern davon, wie dem Propionat, Hydrocortison oder Estern davon, etwa dem Acetat; nicht-steroidalen entzündungshemmenden Arzneimitteln, wie Naproxen, Indomethacin, Diclofenac, Ibuprofen, Dexibuprofen, Ketoprofen, Flurbiprofen, Piroxicam, Tenoxicam, Lornoxicam oder Nabumeton, Phosphodiesterase-4-Hemmern oder p38 MAP-Kinase-Inhibitoren.

22. Zusammensetzung nach Anspruch 21, wobei der zusätzliche Wirkstoff ausgewählt ist unter Betamethason und einem Ester davon, z. B. dem Valerat- oder Dipropionatester.

23. Zusammensetzung nach einem der Ansprüche 1-22 zur Verwendung bei der Behandlung von dermatologischen Erkrankungen oder Zuständen.

24. Zusammensetzung nach Anspruch 23 zur Verwendung bei der Behandlung von dermatologischen Erkrankungen oder Zuständen, die ausgewählt sind unter Psoriasis, Pustulosis palmoplantaris, Ichthyose, Rosacea, Dermatitis und Akne.

## Revendications

1. Composition topique sensiblement anhydre stable au stockage comprenant un mélange homogène de
(a) une quantité thérapeutiquement efficace de calcipotriol sous forme dissoute ;
(b) un mélange de solvants consistant essentiellement en un solvant ester d'acide gras choisi dans le groupe consistant en les esters de glycéryle, par exemple les triglycérides d'acides gras, les esters d'isopropyle d'acides C₁₀₋₁₈ alcanoïques ou alcénoïques, par exemple le myristate d'isopropyle, le palmitate d'isopropyle, l'isostéarate d'isopropyle, le linoléate d'isopropyle, le monooléate d'isopropyle ou les esters de propylène glycol, par exemple le dipelargonate de propylène glycol, ou leurs mélanges ;
un co-solvant alkyl éther gras qui est un composé de formule générale I H(OCH₂C(R²)H)ₓR¹, où R¹ est C₁₋₂₀ alkyle linéaire ou ramifié, chaque R² est individuellement l'hydrogène ou CH₃, et x est un entier de 2-60 ; et
un stimulateur de pénétration lipophile choisi dans le groupe consistant en une N-alkylpipéridone, une N-alkylpyrrolidone, comme la N-méthylpyrrolidone ou une N-hydroxyalkylpyrrolidone, ou le diméthylacétamide, le diméthylsulfoxyde ou la 2-pyrrolidone ;
où le rapport du solvant ester d'acide gras au co-solvant alkyl éther gras au stimulateur de pénétration est dans la plage de 50:25:25 à 75:10:15 ; ledit mélange de solvants étant inclus dans la composition en une quantité qui est suffisante pour dissoudre efficacement ledit calcipotriol, et
(c) un vecteur pharmaceutiquement acceptable sensiblement anhydre.

2. Composition selon la revendication 1, où le solvant ester d'acide gras consiste essentiellement en triglycérides à chaîne moyenne.

3. Composition selon la revendication 1 ou 2, où le co-solvant alkyl éther gras est le polyoxypropylène-15-stéaryl éther, le polyoxypropylène-11-stéaryl éther, le polyoxypropylène-14-butyl éther, le polyoxypropylène-10-cétyl éther, le polyoxypropylène-3-myristyl éther, le polyoxypropylène-5-céteth 20.

4. Composition selon l'une quelconque des revendications 1-3, où ledit mélange de solvants (b) est inclus dans la composition en une quantité dans la plage de 0,1 % à 15 % en poids de la composition, de préférence 1-14 % en poids, 2-12 % en poids ou 5-10 % en poids, comme 10 % en poids de la composition.

5. Composition selon l'une quelconque des revendications 1-4, où le rapport du solvant ester d'acide gras au co-solvant alkyl éther gras au stimulateur de pénétration est 60:15:25.

6. Composition selon l'une quelconque des revendications 1-5 comprenant 0,5-12 % p/p, 1-10 % p/p, 2-8 % p/p, 4-7 ou 5 % p/p ou 6 % p/p du solvant ester d'acide gras.

7. Composition selon l'une quelconque des revendications 1-6 comprenant 0,1-5 % p/p, 0,2-4 % p/p, 1-3 % p/p ou 1,2-2 % p/p comme 1,5 % p/p du co-solvant alkyl éther gras.

8. Composition selon l'une quelconque des revendications 1-7 comprenant 0,1-6 % p/p, 0,3-5 % p/p, 0,5-4 % p/p, ou 1-3,5 % p/p, 2-3 % p/p ou 2,5 % p/p du stimulateur de pénétration.

9. Composition selon l'une quelconque des revendications 1-8 comprenant en outre un agent anti-irritation.

10. Composition selon la revendication 9, où l'agent anti-irritation est le menthol, l'eucalyptol ou le nicotinamide.

11. Composition selon l'une quelconque des revendications 1-10 comprenant en outre un agent capable de neutraliser les impuretés acides ou les produits de dégradation présents dans la composition.

12. Composition selon la revendication 11, où ledit agent est une amine comme la triéthanolamine, le trométamol, la monoéthanol-amine ou la diéthanolamine.

13. Composition selon l'une quelconque des revendications 1-12, où le vecteur comprend au moins une paraffine choisie parmi les paraffines consistant en hydrocarbures ayant des longueurs de chaîne de C₅ à C₆₀, les longueurs de chaîne atteignant un maximum à C₁₄₋₁₆, C₁₈₋₂₂, C₂₀₋₂₂, C₂₀₋₂₆, C₂₈₋₄₀ et C₄₀₋₄₄ (comme déterminé par chromatographie en phase gazeuse), ou leurs mélanges.

14. Composition selon l'une quelconque des revendications 1-13, comprenant en outre un ingrédient augmentant la viscosité.

15. Composition selon la revendication 14, où l'ingrédient augmentant la viscosité est une cire.

16. Composition selon l'une quelconque des revendications 1-15, comprenant en outre une cire de silicone ou une huile de silicone volatile.

17. Composition selon la revendication 16, où l'huile de silicone volatile est le cyclométhicone ou le diméthicone.

18. Composition selon l'une quelconque des revendications 1-17 comprenant 0,001-0,5 mg/g, de préférence 0,002-0,25 mg/g, en particulier 0,005-0,05 mg/g, de calcipotriol.

19. Composition selon l'une quelconque des revendications 1-18 comprenant
0,003-0,008 % p/p de calcipotriol
5-8 % p/p de triglycérides à chaîne moyenne
1-3 % p/p de N-méthylpyrrolidone
1-2 % p/p de polyoxypropylène-15-stéaryl éther
80-90 % p/p de vecteur paraffine.

20. Composition selon l'une quelconque des revendications 1-19, comprenant en outre un ou plusieurs ingrédients thérapeutiquement actifs supplémentaires.

21. Composition selon la revendication 20, où de tels ingrédients actifs supplémentaires sont choisis dans le groupe consistant en les médicaments anti-inflammatoires comme les corticostéroïdes, comme la bétaméthasone et ses esters, par exemple l'ester valérate ou dipropionate, le clobétasol ou ses esters, comme le propionate, l'hydrocortisone ou ses esters, comme l'acétate ; les médicaments anti-inflammatoires non stéroïdiens comme le naproxène, l'indométhacine, le diclofénac, l'ibuprofène, le dexibuprofène, le kétoprofène, le flurbiprofène, le piroxicam, le ténoxicam, le lornoxicam ou la nabumétone, les inhibiteurs de phosphodiestérase 4 ou les inhibiteurs de MAP kinase p38.

22. Composition selon la revendication 21, où l'ingrédient actif supplémentaire est choisi dans le groupe consistant en la bétaméthasone et un ester de celle-ci, par exemple l'ester valérate ou dipropionate.

23. Composition selon l'une quelconque des revendications 1-22 destinée à être utilisée dans le traitement des maladies ou affections dermatologiques.

24. Composition selon la revendication 23 destinée à être utilisée dans le traitement des maladies ou affections dermatologiques choisies dans le groupe consistant en le psoriasis, la pustulose palmoplantaire, l'ichtyose, l'acné rosacée, la dermatite et l'acné.
